**Europäisches Patentamt**

**European Patent Office**

**Office européen des brevets**

(19)

(11) Veröffentlichungsnummer: **0 298 436 B1**

(12) **EUROPÄISCHE PATENTSCHRIFT**

(45) Veröffentlichungstag der Patentschrift: **14.10.92**

(51) Int. Cl.⁵: **A61K 49/02**, A61K 43/00, A61K 37/54

(21) Anmeldenummer: **88110746.0**

(22) Anmeldetag: **06.07.88**

(54) **Verwendung eines Vehikels mit hoher Affinität zu Tumoren.**

(30) Priorität: **08.07.87 DE 3722522**

(43) Veröffentlichungstag der Anmeldung:
**11.01.89 Patentblatt 89/02**

(45) Bekanntmachung des Hinweises auf die
Patenterteilung:
**14.10.92 Patentblatt 92/42**

(84) Benannte Vertragsstaaten:
**AT BE CH DE ES FR GB GR IT LI LU NL SE**

(56) Entgegenhaltungen:
**WO-A-83/03101**
**US-A- 4 663 146**

**CANCER, Band 48, Nr. 6, 1981, American
Cancer Society, US; O.NIKLASSON et al.,
Seiten 1484-1486&NUM;**

**BIOLOGICAL ABSTRACTS, Band 82, Nr. 12,
1986, Biological Abstracts Inc., Philadelphia,
PA (US); M.J.DUFFY et al., Nr. 113600&NUM;**

**BIOLOGICAL ABSTRACTS, Band 66, 01 Oktober 1978, Biological Abstracts Inc., Philadelphia, PA (US); W.L.BIGBEE et al., Seite**

**3999, Nr. 40841&NUM;**

**THE JOURNAL OF NUCLEAR MEDICINE, Band
29, Nr. 7, Juli 1988; S.-L.KARONEN et al.,
Seiten 1194-1198&NUM;**

**KEMIA-KEMI, Band 14, Nr. 11, 1987;
S.-L.KARONEN et al., Seiten 1095-1096&NUM;**

(73) Patentinhaber: **BOEHRINGER INGELHEIM IN-
TERNATIONAL GmbH
Postfach 200
W-6507 Ingelheim am Rhein(DE)**

(72) Erfinder: **Karonen, Sirkka-Liisa, Dr.
Viestitie 6
SF-00370 Helsinki(FI)**
Erfinder: **Lindgren, Jan, Dr.
Aamutie 8A
SF-02210 Espoo(FI)**
Erfinder: **Aronen, Hannu, Dr.
Hiidenkiukaantie 1C 28
SF-00340 Helsinki(FI)**

## Beschreibung

Gegenstand der vorliegenden Erfindung ist die Verwendung eines biologischen Vehikels, bestehend aus radioaktiv markiertem tissue plasminogen activator (tPA), für die Diagnose, Lokalisation und Beobachtung maligner Tumore sowie die Methode zur szintigraphischen Darstellung maligner Neoplasien durch Anwendung dieses Vehikels. Ein therapeutischer Nutzwert wird aufgezeigt.

Das frühzeitige und sichere Auffinden von malignen neoplastischen Geweben und den davon entstandenen Metastasen ist ein entscheidendes Kriterium für die Prognose und für die Heilungsaussicht von bösartigen Tumoren. Neben der Computertomographie spielt in neuerer Zeit die Immunszintigraphie bei der diagnostischen Abklärung von Krebsleiden eine wichtige Rolle. Gerade die letzte Methode, die sich der Anwendung radioaktiv markierter Antikörper bedient, besitzt insbesondere den Nachteil, daß mit einem solchen Antikörper nur eine sehr begrenzte Anzahl an Tumoren verschiedener Gewebe oder nur ein bestimmter Tumortyp erfaßt werden dürfte, da bis heute keine tumorspezifischen Merkmale bekannt sind und praktisch jeder Tumor unterschiedliche Oberflächenantigene trägt.

Die Aufgabe, die dieser Erfindung zugrunde lag, war es, ein für den Menschen verträgliches Vehikel mit hoher Affinität zu Tumorgeweben zu finden, das bei einem breiten Typenspektrum maligner Tumoren sowohl diagnostisch und/oder prognostisch als auch therapeutisch anwendbar ist.

Zur Lösung der Aufgabe wurde überraschenderweise gefunden, daß sich die Verwendung von tPA, einer allelen Variation, eines Derivates oder Fragmentes davon zur Herstellung eines Mittels für die diagnostische und/oder prognostische Abklärung maligner Tumore eignet. Dadurch wird nicht nur die Diagnose von Tumoren verschiedenen Ursprungs ermöglicht, sondern es kann auch ihre Ansprechrate auf eine daraufhin eingeleitete Therapie laufend verfolgt werden. Erfindungsgemäß wird demnach eine gegenüber dem Stand der Technik weniger begrenzt einsetzbare, empfindlichere und schnellere Diagnosemethode bereitgestellt.

Dem Fachmann ist aus immunologischen Gründen bekannt, daß er erfindungsgemäß das Vehikel speziesspezifisch einsetzen wird, beispielsweise humaner tPA beim Menschen, speciesspezifischer tierischer tPA im veterinärmedizinischen Bereich.

Der tPA des Menschen besteht aus einem Polypeptid, welches in einkettiger und zweikettiger Form vorkommt. Das publizierte Molekulargewicht des t-PA liegt, in Abhängigkeit von der Herkunft, im Bereich von 66000 bis 72000 Da. Der N-terminale Teil des tPA Moleküls enthält zwei Strukturen, die den "Kringeln" ähneln bzw. entsprechen, die in Plasminogen und Prothrombin gefunden werden. Darüberhinaus zeigt dieser Teil einen Bereich, der homolog ist mit der "fingerähnlichen" Struktur im Fibronectin (Banyai, L et al. FEBS Letters 163, 37-41, 1983; Pennica, D. et al., Nature 301, 214-221, 1983). Der carboxyterminale Teil des tPA Moleküls zeigt erhebliche Homologie mit anderen Serinproteinasen (Strassburger, W et al, FEBS Letters 157, 219-223, 1983). Neben diesen Kringeln, der fingerähnlichen Struktur und der aktiven Region, gibt es im tPA eine Wachstumsfaktor-Domäne, die mit epidermalem Wachstumsfaktor Homologie zeigt. Somit scheint tPA aus einer Anzahl verschiedener funktioneller Domänen zusammengesetzt zu sein, die jede für sich Homologie mit anderen Proteinen aufweist (Erickson, L.A. et al. Clinics in Haematology 14, 513-530, 1985).

Es ist bekannt, daß tPA eine hohe Affinität zu Fibrin besitzt. Fibrin ist der hauptsächliche Angriffsort für Plasmin bei der Thrombolyse (Wallen, P., Activation of plasminogen with urokinase and tissue activator, in: Thrombosis and urokinase (Paoletti, R., Sherry, S. eds.). Academic Press, London, 91-102, 1977) und ist eine Komponente des Stromas einiger Tumore (Dvorak, H.F. et al., Cancer Metastasis Reviews 2, 41-73, 1983). Auf der anderen Seite variiert der relative Anteil von Stroma und seiner Zusammensetzung von Tumor zu Tumor erheblich.

Aus der WO 83/03101 ist die Verwendung einer pharmazeutischen Zusammensetzung zur Behandlung oder zum Auffinden von Erkrankungen des Blutgerinnungssystems bekannt, welche als wirksames Mittel einen Plasminogen Aktivator enthält, der auch radioaktiv markiert sein kann.

Die US 46 63 146 offenbart eine markierte tPA-Präparation zum Auffinden und Lokalisieren von Blutklumpen.

Maligne Tumore enthalten gegenüber korrespondierenden normalen Gewebe oft mehr Plasminogenaktivität, obwohl erhebliche Variation dieser Aktivität zwischen histologisch gleichen Tumoren besteht (Bigbee, W.L. et al., Biochim. et Biophys. Acta 540, 285-294, 1978; Booth, N.A. et al., Blood 61, 267-275, 1983). Um so unerwarteter war es, daß der Einsatz von radioaktiv markiertem tPA eine neuartige und auf einem neuen Prinzip beruhende Methode eröffnete, eine große Anzahl an Tumortypen autoradiographisch bzw. szintigraphisch zu erfassen und für onkologische Zwecke einzusetzen.

Spekulation ist, das der Plasminogenaktivator bei der Regulation extrazellulärer proteolytischer Aktivitäten eine Rolle spielt. Diese Aktivität könnte für die Infiltration maligner Tumore durch die Basalmembran verantwortlich sein (Gelister, J. S. K. et al., B.M.J. 293, 728-731, 1986). Andererseits gibt es Hinweise, daß nur der Urokinase-ähnliche Aktivator und nicht tPA proteolytische Aktivität besitzt (Danö, K., Urokinase-type plasminogen activator in cancer, XIV annual meeting international society for oncodevelopmental biology and medicine, Helsinki 1986, Abstract 74).

Trotz der noch nicht geklärten Verhältnisse, die bei der Bindung von tPA an Geweben maligner Tumore letztendlich entscheidend sind, zeigen die Resultate der vorliegenden Erfindung überraschend eindeutig, daß radioaktiv markierter tPA bzw. tPA als solcher spezifisch in malignen Tumoren akkumulieren kann.

Somit ist tPA gemäß vorliegender Erfindung ein nützliches Vehikel für einen zielgerichteten Transport geeigneter Markersubstanzen und als spezifisches Mittel sowohl für biochemische und pathologisch-diagnostische als auch prognostische Untersuchungszwecke bei mutmaßlichen oder vorbestehenden malignen Tumoren einsetzbar. Beispielsweise kann der gemäß vorliegender Erfindung anzuwendende tPA nach bekannten Methoden mit bekannten physiologisch unbedenklichen oder vorteilhaften Radioisotopen markiert werden. Als besonders vorteilhafte Radioisotope sind diejenigen des Jods zu nennen (z.B. $^{131}$J, $^{123}$J). Vorteilhafterweise liegt der einzusetzende Radioaktivitätsdosisbereich für $^{131}$J zwischen 10 MBq und 500 MBq, insbesondere zwischen 40 MBq und 230 MBq und für $^{123}$J zwischen 10 MBq und 1000 MBq, insbesondere zwischen 74 und 370 MBq. Weiterhin können beispielsweise auch $^{99m}$Tc oder $^{111}$In oder aber chelatgekoppelte Metallionenisoptope zur Markierung eingesetzt werden.

Radioaktiv markierter tPA kann somit für die Ganz- oder Teilkörperszintigraphie eingesetzt werden, nachdem er in einem Patienten systemisch (z.B. intravenös) oder lokal (d.h. in die Nähe eines vermuteten Tumorgeschehens, z.B. im.) injiziert worden ist. Nach der Applikation in einen Patienten wird es ermöglicht, die genannten diagnostischen Parameter eines maligne entarteten Gewebes, insbesondere solches mit Stromaanteil (z.B. solide Tumore), zu bestimmen. Auch kann radioaktiv markierter tPA zur Prognose eines vorbestehenden Tumors und seiner Entwicklung, vor einer, im Laufe einer oder nach einer therapeutischen Behandlung benutzt werden.

Der erfindungsgemäß zu verwendende tPA kann über bekannte konventionelle Zellkulturen tierischen bzw. menschlicher Herkunft oder durch bekannte gentechnologische Verfahren, d.h. mit den bekannten Methoden der DNA-Rekombination, hergestellt werden. Bekannterweise eignen sich für das zuletzt genannte Verfahren sowohl prokaryotische als auch eukaryotische Zellen.

Bevorzugt wird die Verwendung von tPA oder von Fragmenten davon, welche durch DNA-Rekombination erhalten werden können. Beispielsweise kann nach der EPA 117059, nach der DEO 3316297 oder nach Collen, D., Nature 301, 214-221, 1983, verfahren werden.

Unter tPA wird der durch genetisch manipulierte Mikroorganismen oder durch entsprechend manipulierte oder natürliche Zellkultursysteme gebildete Plasminogen-Aktivator vom Gewebetyp verstanden.

Für den Durchschnittsfachmann ist bekannt, daß natürliche allele Variationen individuumspezifisch vorkommen und sich durch eine oder mehrere unterschiedliche Aminosäuren oder durch unterschiedliche Nukleotide bzw. DNA-Sequenzen manifestieren. Derartige Variationen oder Mutationen, die auch durch die bekannten Methoden der DNA-Rekombination bzw. durch gezielte Mutagenese erzeugt werden können, wie sie beispielsweise in der EPA 93619 oder EPA 199 574 beschrieben werden, umfassen einfache odere mehrfache Substitutionen, Deletionen, Additionen, Insertionen oder Inversionen. Umfaßt werden auch tPA-Derivate, die den für tPA charakteristischen "Kringel"-Bereich und Serinproteasebereich enthalten, ansonsten aber durch eine der vorstehend beschriebenen Weisen verändert sind. Es fallen ferner auch solche tPA-Derivate unter die erfindungsgemäße Verwendung, die die charakteristische, erfindungsgemäße Aktivität oder Affinität aufweisen oder eine gegenüber der genuinen Form höhere Aktivität oder Affinität zeigen (z.B. EPA 93619, EPA 199574).

Darüberhinaus kann nicht nur radioaktiver tPA als ganzes Molekül für die geschilderten Zwecke eingesetzt werden, sondern auch jedwede an malignes Gewebe oder an das Stroma maligner Tumore bindende Teile oder Fragmente davon oder in der bekannten Zusammensetzung veränderte Moleküle oder chimäre Moleküle des tPA.

Völlig überraschend wurde nämlich gefunden, daß nicht nur das komplette tPA-Molekül an Tumore bindet, sondern daß ein über HPLC isoliertes ca. 10 kDa großes Fragment davon eine hohe Affinität zu Tumorgeweben aufweist. Das radioaktiv markierte ca. 10 kDa Fragment des tPAs zeigte gegenüber dem nativen tPA-Molekül sogar eine sehr viel stärkere Akkumulation im Tumorgewebe. Es war ferner sehr überraschend, daß dieses ca 10 kDa Fragment offenbar an Tumororte bindet, welche keine oder nur geringe Fibrinanteile enthalten. Der Nachweis dafür konnte beispielsweise durch Doppelmarkierung mit $^{111}$In-Antifibrin und $^{131}$I-rtPA erhalten werden, wonach $^{131}$I-tPA im peripheren Teil des Tumors bindet, nicht aber im nekrotischen, Fibrin enthaltenden Zentrum des Tumors, dem Bindungsort von $^{111}$In-Antifibrin.

Z.B. sind daher auch solche veränderten tPA Moleküle oder Fragmente davon von Vorteil gegenüber dem proteolytisch wirksamen tPA, die keine Proteolyseaktivität mehr besitzen aber dennoch hohe Affinität zu Tumorgeweben zeigen.

Die Verwendung derartiger "second-generation" tPAs ist ebenfalls Bestandteil dieser Erfindung. Da die Struktur des tPAs bekannt ist, können durch "protein engineering" tPA-Derivate hergestellt werden, die beispielsweise mehrere Finger - und/oder epidermale Wachstumsfaktor - Domänen aufweisen oder nur aus diesen Domänen bzw. dem N- Terminus bestehen.

Hinsichtlich einer gezielten und effektiven therapeutischen Behandlung maligner Tumore steht der Fachmann heute noch vor unüberwindlichen Schwierigkeiten. Obwohl in letzter Zeit monoklonale Antikörper gegen Tumore entwickelt worden sind, auch als Vehikel in Konjugation mit cytostatischen Substanzen - bekannt als Immunotoxine - (z.B. Bjorn, M.J., Cancer Res. 46, 3262-3267, 1986), lassen selbst diese modernen Therapieversuche die ungelösten Probleme bestehen. Es bleibt nämlich nach wie vor unbeantwortet, wie eine gewünschte Bindungseffizienz an die Zelloberfläche zu erreichen ist, wie Neutralisierungseffekte zu vermeiden sind (z.B. Bjorn, M.J., loc. cit.), wie die verschiedensten Tumortypen, insbesondere mit heterologer Zellpopulation, in toto zu erreichen sind, wie niedrige Bindungsaffinitäten von konjugierten monoklonalen Antikörpern bzw. Immunotoxinen und deren Spezifität für die Tumorzellen zu erhöhen sind.

Völlig unklar ist auch, ob derartige Immuntoxine bei soliden Tumoren mit dichtem Bindegewebsanteil wirksam sein können, da die Antikörper vorzugsweise an Oberflächenantigene binden und nicht innerhalb des Tumors gelegene Zellmassen bzw. das Tumorzentrum erreichen. Viele dieser ungelösten Probleme sind dem Fachmann bekannt (z.B. Vitetta, E.S., Science, 219, 644-650, 1983).

Dadurch, daß die Erfinder der vorliegenden Patentanmeldung die überraschende Affinität von tPA oder insbesondere eines seiner Fragmente zu Tumoren erkannt haben, wird die Fachwelt auch um eine unerwartet neue Möglichkeit, Tumore gezielt zu therapieren, bereichert. Dementsprechend ist tPA oder wesentliche Teile davon für die erfindungsgemäße Anwendung als Vehikel zu verstehen, welches nicht nur zur diagnostischen und/oder prognostischen Abklärung maligner Tumore von Nutzen ist, sondern auch therapeutische Bedeutung erlangt, da tPA, gekoppelt mit einem dem Fachmann bekannten cytostatisch oder cytocidal, d.h. gegen Tumorzellen wirkenden synthetischen oder natürlichen Agens (z.B. Ricin oder andere Pflanzentoxine, Antikörper, Hormone, Translations-Inhibitoren oder Immunmodulatoren bzw. Teilen davon), dieses gezielt an den Ort eines Tumorgeschehens transportieren kann. Insbesondere sind dazu tPA-Fragmente mit hoher Affinität zu Tumoren geeignet, wie vorzugsweise das 10 kDa Fragment des tPA-Moleküls, welches durch HPLC isoliert werden konnte. Zum Zweck der Realisierung einer solchen therapeutischen Nutzung sind dem Fachmann hinreichend bekannte und etablierte Verfahren bekannt, um z.B. mit Toxinen oder Haptenen konjugierte Proteine herstellen zu können (WO 85/03508; Varga, J.M., in: Methods in Enzymology, 112, 259-269, 1985; Bacha, P. et al., J. Biol. Chem. 258, 1565-1570, 1983; Gendloff, E.H. et al., J. Immunol. Methods 92, 15-20, 1986; Duncan, R.J.S. et al., Anal. Biochem. 132, 68-73, 1983; King, T.P. et al., Biochemistry 25, 5774-5779, 1986; Myers, D.A. et al., Biochem. J. 227, 343, 1985, um nur einige zu nennen). Maßnahmen, die geeignet sind, tPA oder wesentliche Fragmente davon mit den gegen Tumorzellen wirkenden Toxinen zu konjugieren, liegen somit im Bereich des Fachwissens des Fachmannes.

Ebenfalls eignet sich radioaktiv markierter tPA oder wesentliche Fragmente davon. Beispielsweise kann $131_I$ markierter tPA oder wesentliche Teile davon oberhalb einer Radioaktivitätsdosis von 500 MBq therapeutisch eingesetzt werden.

Da beispielsweise langsamwachsende solide Tumore einer konventionellen Therapie schwer zugänglich sind, weil solche Therapien gegen sich teilende Zellen gerichtet sind, können z.B. derartige Tumore geeignete Kandidaten für eine Therapie mit einem konjugierten tPA Molekül oder einem konjugierten Teil des tPAs als Vehikel darstellen. In einem solchen Fall läge der Vorteil beispielsweise darin, daß tPA am Tumor für relativ lange Zeit gebunden (akkumuliert) bleibt und das Konjugat als cytotoxisches Agens laufend und quasi topisch auf das Tumorgewebe gezielt einwirkt. Eine Progression oder Exazerbation im Tumorwachstum durch (wieder) einsetzende Mitoseaktivität kann demzufolge durch das am Ort des Tumorgeschehens befindliche Therapeutikum unterdrückt werden.

Somit eröffnen sich mit dieser Erfindung neue Möglichkeiten der Krebsdiagnose und Krebstherapie.

In einer Studie wurden 11 männliche und 4 weibliche Patienten im Alter von 32 bis 82 Jahren (Mittel 58) unter Verwendung von $^{131}I$ markiertem tPA untersucht. Die Markierung mit $^{131}I$ erfolgte nach bekannten Methoden. Alle Patienten hatten maligne Tumore oder Metastasen, die histologisch oder radiologisch (röntgenologische Computertomographie) verifiziert wurden. Die angewandte Radioaktivitätsdosis lag im Bereich von 40 bis 230 MBq (im Mittel 165 MBq). Nach Blockierung der Aufnahme des Radioisotops des Jods durch die Schilddrüse mit oral verabreichtem Natriumjodid und Perchlorat (tägliche Dosis von 420 mg bzw. 800 mg für 10 Tage), erhielten die Patienten eine intravenöse Injektion von $^{131}I$-tPA. Die Verteilung der Radioaktivität wurde mit einer large-field-of-view Gamma-Kamera (Kristalldurchmesser am Kopf der Kamera von 40 cm oder mehr) 4 und 24 Stunden nach der Injektion untersucht, in drei Fällen wurde die Darstellung mit der Gamma-Kamera 2 bis 5 Tagen nach der Injektion wiederholt. Die Computeranalyse der erhaltenen Bilder wurde zur Bestimmung des "target-to-nontarget" Verhältnisses und zur Berechnung der prozentualen Aufnahme der Dosis im Zielgebiet durchgeführt.

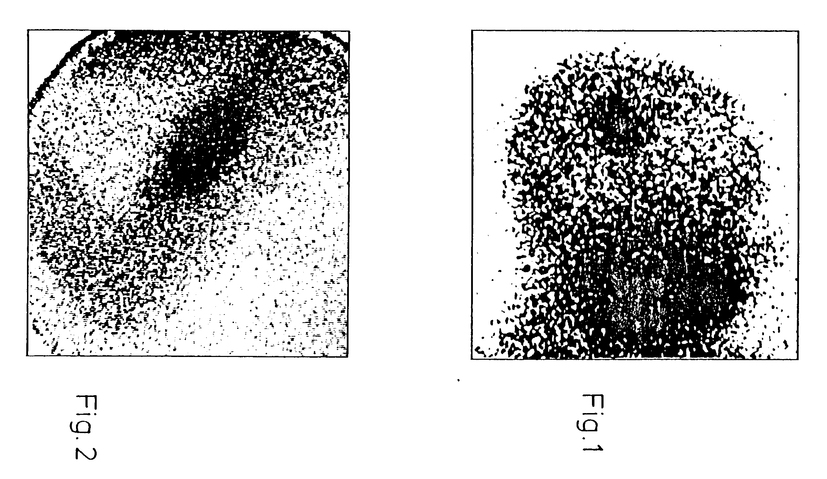

Die von den 15 Patienten mit malignen Tumoren oder mit Metastasen erhaltenen szintigraphischen Ergebnisse mit $^{131}$I-tPA sind in nachstehenden Beispielen aufgeführt. Die relative Intensität der Radioaktivitätsakkumulation wurde nach subjektiver Beurteilung in die Kategorien "leicht", "mittel" oder "stark" unterteilt. In 10 Fällen wurde eine Akkumulation von $^{131}$I-tPA festgestellt. Bei drei Patienten betrug die verabreichte Dosis weniger als 100 MBq; mit negativem Ergebnis in den Abbildungen. Zwölf Patienten erhielten eine Dosis größer als 100 MBq. In dieser Gruppe wurde bei 11 Patienten eine Akkumulation von radioaktivem tPA im krankhaft veränderten Gewebe gefunden. Bei vier Patienten erfolgte eine cerebrale Anreicherung von radioaktivem tPA und bei acht Patienten in extracerebralen Bereichen. Die Abbildungen 1 und 2 zeigen ein typisches Ergebnis. Drei Patienten zeigten sowohl cerebrale als auch extracerebrale Akkumulation von $^{131}$I-tPA. Bei fünf Patienten wurde keine pathologische Anreicherung von $^{131}$I-tPA beobachtet. In dieser Gruppe waren zwei Adenokarzinome, ein Hypernephrom, ein Prostatakarzinom und ein Harnblasenkarzinom diagnostiziert worden.

Alle Akkumulationen waren 24 Stunden und, außer in zwei Fällen, 4 Stunden nach der Injektion von $^{131}$I-tPA sichtbar. Die "target-to-nontarget" Verhältnisse zum Zeitpunkt 24 Stunden nach der Injektion, die durch Computeranalysen der dafür aufbewahrten Bilder bestimmt wurden, variierten von <1,2:1 bis 2.1:1. Bei drei untersuchten Patienten war auch fünf Tage nach der Injektion von $^{131}$I-tPA eine Akkumulation festzustellen.

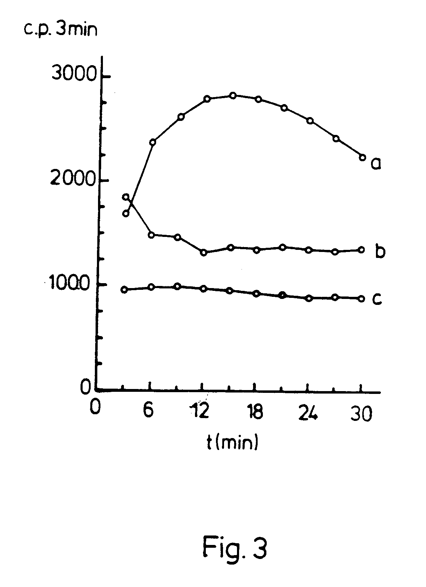

Die Bioverteilung von $^{131}$I-tPA zeigt Fig. 3. Drei Minuten nach Injektion von $^{131}$I-tPA erfolgt eine Anreichung in der Leber von 59% und in der Milz von 96% der maximalen Radioaktivität im jeweiligen Organ. In der Leber wurde die maximale Radioaktivität nach 15 min und in der Milz nach 9 min erreicht. Nach Erreichen des Maximums wurden innerhalb der nächsten 15 min 20% der Radioaktivität in der Leber und 10% der Radioaktivität in der Milz eliminiert. Nach 24 Stunden war die Radioaktivität in der Leber 2% und in der Milz <1% des Maximalwertes.

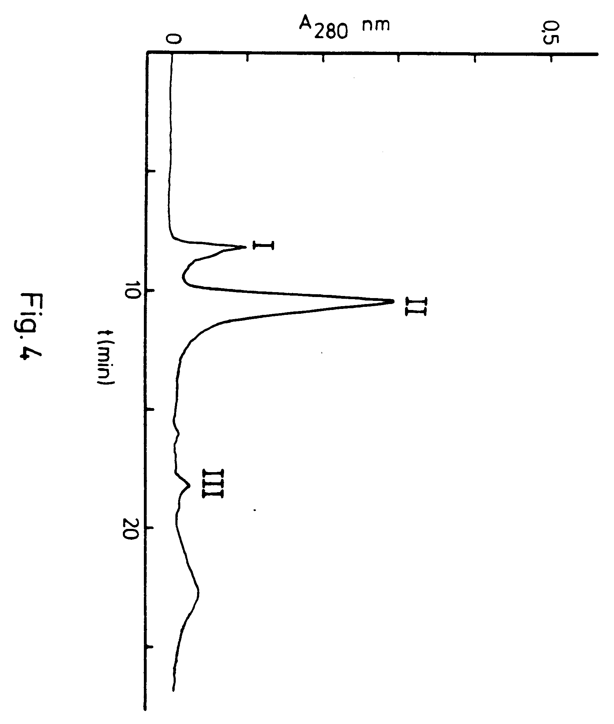

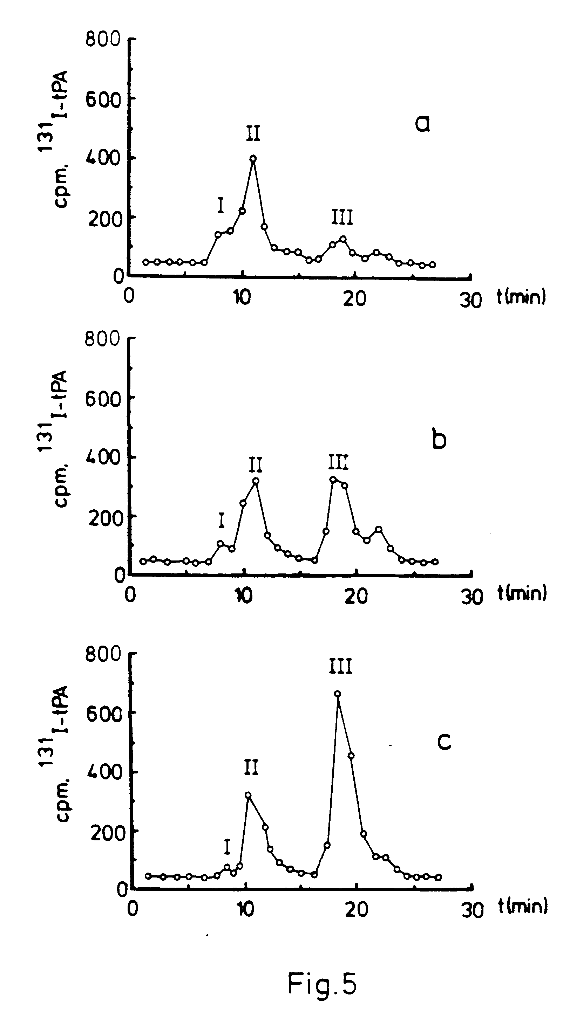

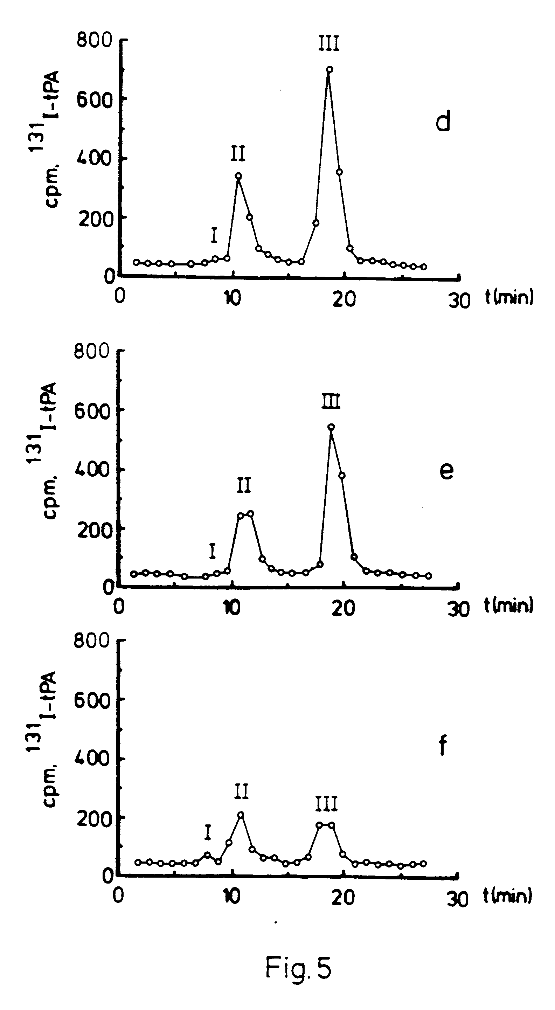

In Blutproben konnten über HPLC drei Fraktionen von $^{131}$I-markiertem tPA gefunden werden, die mit I, II und III bezeichnet wurden (Fig. 4). Die annähernden Molekulargewichte der einzelnen Fraktionen, die mit Hilfe von Markerproteinen bestimmt wurden, sind: I = 600 kDa, II = 80 kDa, III = 10 kDa. Die Plasmaradioaktivität fiel im selben Maß wie bei der radioaktiv markierten Proteinkomponente II (80 kDa). Vor dieser Komponente II wurde ein größerer Komplex I (600 kDa) gefunden, der höchstwahrscheinlich einen $^{131}$I-tPA-Plasminogen-Aktivator Inhibitor (PAI) - Komplex darstellt. Die kleinste und, wie vorher beschrieben, für den erfindungsgemäßen Gegenstand interessanteste gefundene Komponente III mit ca. 10 kDa hatte nur eine minimale zellgebundene Aktivität. Sie betrug nur 0.35% der gesamten Blutradioaktivität. Sie wurde in Blutplättchen (platelet) und in der Lymphocyten/Monocyten-Fraktion gefunden, nicht aber in der Erythrozyten-Fraktion. Daraus sind folgende Schlußfolgerungen zu ziehen: 1. $^{131}$I-tPA wird schnell an seine Träger-Proteine gebunden und zu einer kleineren Komponente von ca. 10 kDa abgebaut, wobei die Radioaktivität, die im Elutionsvolumen gefunden wird, schnell und im gleichen Maße ansteigt wie die der anderen Komponenten absinkt (Fig. 5). 2. Höchstwahrscheinlich repräsentiert die duch malignes Gewebe akkumulierte Radioaktivität einen solchen 10 kDa Metaboliten von $^{131}$I-tPA, der gegenüber dem ganzen tPA-Molekül ein ideales Vehikel im Sinn der vorliegenden Erfindung darstellt. Unerwünschte Nebenwirkungen nach Gabe des radioaktiv markierten tPAs wurden nicht beobachtet.

Legenden zu den Abbildungen

Fig.1    zeigt starke Akkumulation von $^{131}$I-tPA in einer Hirnmetastase eines Lungenkarzinoms.

Fig.2    zeigt starke Anreicherung von $^{131}$I-tPA in einer Metastase eines Lungenkarzinoms im linken oberen Arm.

Fig.3    Zeit-Aktivitätskurven von Leber, Herz und Milz nach intravenöser Injektion von $^{131}$I-markiertem tPA (190 MBq) bei einem männlichen Patienten a = Leber, b = Herz, c = Milz

Fig.4    Gelfiltrations-Chromatographie von Plasma, 15 min. nach Injektion von $^{131}$I-tPA in Patient 1 (Beispiel 1). I = 600 kDa, II = 80 kDa, III = 10 kDa.

Fig.5    HPLC-Läufe von Plasmaproben nach Injektion von $^{131}$I-tPA in Patient 1 (Beispiel 1), entnommen nach verschiedenen Zeitintervallen.
a = nach 15 min, b = nach 30 min, c = nach 60 min, d = nach 150 min, e = nach 240 min, f = nach 24 Stunden

Die folgenden Beispiele sollen die Erfindung näher erläutern, aber nicht einschränken. Die Unterteilung der Intensität der relativen Aufnahme von $^{131}$I-tPA (Akkumulation von $^{131}$I-tPA in verschiedenen malignen Primär- und Sekundärtumoren) erfolgte anhand der Szintigramme in den Kategorien schwach (1 +), mittel (2 +) und stark (3 +). Die einzelnen Daten über die verschiedenen bestimmten Parameter sind in der nach den Beispielen angegeben Tabelle 1 aufgeführt.

Beispiel 1:

Weiblicher Patient, 38 Jahre, mit Karzinom in der rechten Brust sowie metastasischem Pleuraerguß in beiden Lungen, einer großen Metastase in der Leber und zwei Metastasen im rechten Ovar.

Beispiel 2:

Männlicher Patient, 66 Jahre, mit drei Jahre vorher diagnostiziertem Prostatakarzinom. Das Anfangstumorstadium (initial staging) war T3 NX MO, eine Orchiektomie und Elektroresektion der Protasta wurde durchgeführt. Der Patient hatte Metastasen im Gebiet der linken Clavicula und ein nach Bestrahlung aufgetretenes Rezidiv eines 13 cm durchmessenden Tumors in der Nähe der Prostata.

Beispiel 3:

Männlicher Patient, 65 Jahre, bei dem sechs Monate vorher ein kleinzelliges Lungenkarzinom entdeckt wurde, das chemotherapeutisch behandelt wurde. Nach der Chemotherapie wurde die Szintigraphie durchgeführt. Zu diesem Zeitpunkt hatte der Patient Tumorrezidive in den Lungen und im Mediastinum und Hirnmetastasen.

Beispiel 4:

Männlicher Patient, 73 Jahre, der vor 11 Jahren wegen eines Hypernephroms operiert worden ist. Acht Jahre später wurden Lungenmetastasen inclusive einer pituitären Veränderung festgestellt. Die Szintigraphie erfolgte nach Radiotherapie des pituitären Bereichs

Beispiel 5:

Männlicher Patient, 32 Jahre, mit einem vier Jahre vorher operiertem malignen Melanom der Haut. Zum Zeitpunkt der Szintigraphie wurden mehrere subcutane und zwei Hirnmetastasen gefunden. Der Patient erhielt Interferon und eine regionale Strahlentherapie.

Beispiel 6

Männlicher Patient, 70 Jahre, mit einem neun Monate vorher erkanntem Adenokarzinom im linken Lungenhilus, das radiotherapeutisch behandelt wurde. Das CT ergab eine Infiltration des Mediastinums, von Metastasen befallene Lymphknoten der retrosternalen und prätrachealen Regionen und eine Hirnmetastase.

Beispiel 7:

Weiblicher Patient, 82 Jahre, mit Harnblasenkarzinom, das 10 Monate vorher diagnostiziert wurde. Strahlentherapie im Beckenbereich wurde durchgeführt. Zur Untersuchungszeit hatte der Patient diffuse Lungenmetastasen und Lebermetastasen.

Beispiel 8:

Männlicher Patient, 69 Jahre, mit kleinzelligem Lungentumor, der 13 Monate vorher diagnostiziert und der chemotherapeutisch und durch Strahlentherapie des Medistinums behandelt wurde. Im CT waren zwei cerebrale Metastasen zu erkennen. Der Patient hatte auch Lebermetastasen, die durch sonographische Untersuchung verifiziert wurden.

Beispiel 9

Männlicher Patient, 37 Jahre, bei dem ein Neuroepitheliom vom Schädel 27 Monate vorher entfernt wurde. Eine neue Läsion wurde aus dem Winkel des linken Kiefers 23 Monate später exzidiert. Zwei Monate vor der Szintigraphie trat ein Rezidiv im linken oberen Nacken auf. Der Patient erhielt eine Chemotherapie.

Beispiel 10:

Männlicher Patient, 81 Jahre, mit einem Tumor im hinteren Teil der rechten Pleura und Pleuraerguß. Die Größe des Tumors betrug 6 x 12 cm. Die Diagnose eines malignen fibrotischen Histiocytoms wurde anhand einer Biopsieprobe, gestellt. Der Patient erhielt vor der Szintigraphie keine Therapie.

Beispiel 11

Männlicher Patient, 36 Jahre, mit einem Magenkarzinom, das nach der Operation chemotherapeutisch behandelt wurde. Wegen eines Magentumors wurde eine palliative Gastrektomie fünf Monate vor der Szintigraphie durchgeführt. Zum Zeitpunkt der Szintigraphie wurden im Bereich der linken Clavicula vergrößerte Lymphknoten gefunden. Durch CT wurden bei diesem Patient auch Metastasen in der Leber, in der linken Brustwand und in den paraaortischen Lymphknoten nachgewiesen.

Beispiel 12:

Weiblicher Patient, 47 Jahre, bei der 2 Jahre und 10 Monate vorher ein 4mm großes Melanom vom rechten Fuß entfernt wurde. Von Metastasen befallene Lymphknoten in der rechten inguinalen Region wurden gefunden. Zwei Metastasen traten später im linken Bein auf. Diese Bereiche wurden bestrahlt. Zum Zeitpunkt der Szintigraphie konnten einige subcutane Metastasen, einschließlich einer 6 x 4cm großen Metastase in der linken Brustwand gefunden werden. Die Patientin wurde chemotherapeutisch behandelt.

Beispiel 13:

Männlicher Patient, 48 Jahre, mit schuppenzelligen Ösophagustumor und großen Lymphknotenmetastasen auf beiden Seiten des Halses. Die Lymphknotenmetastasen wurden vor der Szintigraphie strahlentherapeutisch behandelt. Aktives Tumorgewebe wurde durch Autopsie der zervikalen Lymphknoten gefunden.

Beispiel 14:

Männlicher Patient, 63 Jahre, bei dem 8 Monate vorher ein schuppenzelliger Lungentumor operiert wurde. Durch CT konnte eine Cerebral-Metastase vier Monate später nachgewiesen werden und der Patient erhielt eine Strahlentherapie. Zum Zeitpunkt der Szintigraphie hatte der Patient Metastasen im linken oberen Arm und in der Brustwand.

Beispiel 15

Weiblicher Patient, 65 Jahre, mit zwei Malignitäten: Brustkrebs und Krebs des Rectums, die beide vier Jahre vorher operiert worden waren. Die Patientin wurde wegen Lungenmetastasen ins Hospital überwiesen. Die Metastasen waren wahrscheinlich rectalen Ursprungs, da im CT Tumorreste im Bereich des Rectums aufgefunden wurde.

Beispiel 16

Applikation von radioaktivem tPA

a. 0.1 mg tPA wurde nach der bekannten Jodogen-Methode mit $^{131}$I und einer Aktivität von 110 MBq radioaktiv markiert (Fraker P.J., Speck, J.C., Biochem. Biophys.Res.Comm. 80, 849-857, 1978) und in blutisotonischer wässeriger Lösung aufgenommen. Nach Sterilfiltration wurde diese Dosis von radioaktiv markiertem tPA intravenös in Patienten injiziert, die an malignen Tumoren (Melanom, Adenokarzinom des Colons oder der Brustdrüse oder epidermoides Lungenkarzinom) litten. Die externen Ganzkörperszintigraphien wurden mit einer Gammakamera 1, 2, 4 und 7 Tage nach der Injektion durchgeführt.
b. 1,0 mg tPA, radioaktiv mit $^{131}$I markiert mit einer Aktivität von 185 MBq, wurde wie unter Beispiel 16a aufgearbeitet, ebenso bei gleichen Malignitäten appliziert. Die Ganzkörperszintigraphie erfolgte wie unter Beispiel 16a angegeben.

Beispiel 17

Bioverteilung von $^{131}$I-tPA

190 MBq von $^{131}$I-tPA wurden intravenös in einen 22-jährigen männlichen Patienten mit behandeltem "low-grade" Astrozytom injiziert. Die Erfassung der Radioaktivität in Leber, Milz uns Herz erfolgte dynamisch. Fig. 3 zeigt die erhaltenen Zeit-Aktivitätskurven für die jeweiligen Organe Leber, Herz und Milz.

Beispiel 18

Identifizierung des ca. 10 kDa tPA Fragments

In einer Dosis von 3-5 mCi/mg Protein wurde $^{131}$I-markiertes rekombinantes tPA ($^{131}$I-rtPA) in Patienten injiziert und Blutproben von 9 Patienten getestet. Proben wurden vor der i.v. Injektion sowie 15, 30, 60, 150, 240 min und 24 Stunden nach der Injektion entnommen und nach bekannter Weise in EDTA-Röhrchen gegeben. Plasma und Blutzellen wurden danach sofort durch Zentrifugation getrennt. Blutplättchen und die Lymphocyten/Monocyten-Fraktion wurden nach bekannten Methoden mittels LEUKOPREP$^R$ - Röhrchen getrennt. Die HPLC Ausschlußchromatographie (Gelfiltration des Plasmas) wurde unter folgenden Bedingungen durchgeführt:

| | |
|---|---|
| Apparatur : | LKB 2150 HPLC Pump + LKB 2152 Controller |
| UV-Detektor: | LKB 2151, variabler Wellenlängen Monitor |
| Vorsäule: | Ultropac TSK SWP (7.5 x 75mm) LKB |
| Säule : | Ultropac TSK G300SW (7.5 x 300mm) LKB |
| Fraktionskollektor : | LKB 2212 Helirac |
| HPLC-Puffer: | 20 mmol/l L-Arginin, 11 mmol/l Phosphorsäure, 0.05% Natriumazid, pH 7.3 |
| Fließgeschwindigkeit: | 0.75 ml/min, 1-Minuten-Fraktionen wurden gesammelt |
| Wellenlänge: | 280 nm |
| Gammadetector: | Beckman Model 170 Radioisotope Detector und Compugamma Modell 1282, LKB Wallac |
| Proben: | Die Plasmaproben wurden 1:10 mit HPLC-Puffer verdünnt und vor der Injektion auf die Säule filtriert |
| Gelfiltration: | Die Absortion des Eluates wurde bei 280 nm aufgezeichnet und die Radioaktivität außerdem an einem Gamma-Zähler gemessen |

EP 0 298 436 B1

Tabelle 1: Akkumulierung von $^{131}$I-tPA in verschiedenen malignen Primär- und Sekundärtumoren. Die Intensität der relativen Aufnahme von $^{131}$I-tPA wurde anhand der erhaltenen Szintigramme mit schwach (1+), mittel (2+) und stark (3+) unterteilt.

| Beispiel Nr. (Patient) | Alter | Geschlecht | Diagnose | $^{131}$I-tPA Dosis [MBq] | Intensität (rel. Aufnahme von $^{131}$I-tPA | target-to-non target Verhältnis | Zeit nach Injektion von $^{131}$I-tPA [h] |
|---|---|---|---|---|---|---|---|
| 1 | 38 | F | Brustkrebs | 37 | negativ | – | – |
| 2 | 66 | M | Metastasiertes Prostatakarzinom | 181 | negativ | – | – |
| 3 | 65 | M | Kleinzelliges Lungenkarzinom; mit Hirnmetastasen | 200 | 1+ 1+ | 1,3 1,3 | 4 24 |
| 4 | 73 | M | Hypernephrom | 100 | negativ | – | – |
| 5 | 32 | M | Malignes Melanom; mit 2 Metastasen | 155 | 1+, 2+ | 1.3 1.5 1.3 | 4 24 48 |

| Beispiel Nr. (Patient) | Alter | Geschlecht | Diagnose | $^{131}$I-tPA Dosis [MBq] | Intensität (rel. Aufnahme von $^{131}$I-tPA) | target-to-non target Verhältnis | Zeit nach Injektion von $^{131}$I-tPA [h] |
|---|---|---|---|---|---|---|---|
| 6 | 70 | M | Bronchialkarzinom; | 174 | 1+ | | |
| | | | Hirnmetastase | | 1+ | 1.3 | 4 |
| 7 | 82 | F | Harnblasenkarzinom; diffuse Lungenmetastasen | 67 | negativ | - | - |
| 8 | 69 | M | kleinzelliges Lungenkarzinom; | 233 | | | |
| | | | Hirnmetastasen | | 2+, 3+ | 1.4 | 4 |
| | | | | | | 1.7 | 24 |
| 9 | 37 | M | Neuroepitheliom am Nacken | 181 | 2+ | 1.4 | 4 |
| 10 | 81 | M | Histocytom der Pleura | 192 | 1+ | 1.2 | |
| 11 | 36 | M | Adenokarzinom des Magens; Metastase in der linken Brustwand | 185 | 1+ | 1.2 | |

| Beispiel Nr. (Patient) | Alter | Geschlecht | Diagnose | $^{131}$I-tPA Dosis [MBq] | Intensität (rel. Aufnahme von $^{131}$I-tPA) | target-to-non target Verhältnis | Zeit nach Injektion von $^{131}$I-tPA [h] |
|---|---|---|---|---|---|---|---|
| 12 | 47 | F | Malignes Melanom; Metastase in der linken Brustwand | 229 | 3+ | 1.3 | 24 |
| 13 | 48 | M | Schuppenzellkarzinom des Ösophagus; Metastasen im linken Nacken | 185 | 3+ | 1.7 | 24 |
| 14 | 63 | M | Lungenkarzinom; Metastase im linken oberen Arm | 185 | 3+ | 1.4 / 2.1 | 4 / 24 |
| 15 | 65 | F | Multiple Lungen-metastasen; Primärtumor unbekannt | 215 | 1+ | 1.2 | 24 |

## Patentansprüche

1. Verwendung von tPA, einer allelen Variation, eines Derivates oder Fragmentes davon, zur Herstellung eines Mittels für die diagnostische und/oder prognostische Abklärung maligner Tumore.

**2.** Verwendung nach Anspruch 1, dadurch gekennzeichnet, daß der darin enthaltene tPA oder ein Fragment davon natürlichen Ursprungs sind und aus eukaryotischen Zellkulturen isoliert wurden.

**3.** Verwendung nach Anspruch 1, dadurch gekennzeichnet, daß der darin enthaltene tPA oder ein Fragment davon aus durch DNA-Rekombination veränderten prokaryotischen und eukaryotischen Zellen entstammen.

**4.** Verwendung nach Ansprüchen 1 bis 3, dadurch gekennzeichnet, daß der darin enthaltene tPA oder ein Fragment davon im hauptsächlich aus den tumorbindenden Anteilen bestehen und keine Proteolyseaktivität zeigen.

**5.** Verwendung nach einem der Ansprüche 1 bis 4, dadurch gekennzeichnet, daß der darin enthaltene tPA oder ein Fragment davon durch gezielte Mutagenese veränderte Derivate sind, welche eine gegenüber den ursprünglichen, genuinen Formen zumindest gleiche charakteristische Aktivität oder Affinität zu Tumorgeweben aufweisen.

**6.** Verwendung nach einem der Ansprüche 1 bis 5, dadurch gekennzeichnet, daß der darin enthaltene tPA oder ein Fragment davon aus durch multiple Allelie oder Polymorphismen bedingten Variationen vorliegt.

**7.** Verwendung nach einem der Ansprüche 1 bis 6, dadurch gekennzeichnet, daß der darin enthaltene tPA eine allele Variation, ein Derivat oder Fragment davon ist, welches mindestens eine Finger- und/oder epidermale Wachstumsfaktordomäne besitzt oder nur aus je mindestens einem dieser N-terminalen Teile oder aus Kombinationen dieser Teile besteht.

**8.** Verwendung nach einem der Ansprüche 1 bis 7, dadurch gekennzeichnet, daß das tPA Fragment in der Größenordnung von 10 kDa vorliegt.

**9.** Verwendung nach einem der Ansprüche 1 bis 8, dadurch gekennzeichnet, daß der tPA oder ein Derivat davon markiert sind.

**10.** Verwendung nach einem der Ansprüche 1 bis 9, dadurch gekennzeichnet, daß der darin enthaltene tPA, eine allele Variation, ein Derivat oder ein Fragment davon, radioaktiv markiert sind, insbesondere mit $^{131}$I, $^{123}$I, $^{99m}$Tc und/oder $^{111}$In

**11.** Verwendung von tPA, einer allelen Variation, eines Derivates, eines Konjugates oder eines Fragmentes davon zur Herstellung von Arzneimitteln für die therapeutische Behandlung von malignen Tumoren.

**12.** Verwendung nach Anspruch 11, dadurch gekennzeichnet, daß das tPA, eine allele Variation, ein Derivat oder Fragment davon enthaltende Arzneimittel als Konjugat mit mindestens einer cytotoxisch wirksamen Substanz vorliegt.

**13.** Verwendung nach Anspruch 12, dadurch gekennzeichnet, daß der im Arzneimittel enthaltene tPA, eine allele Variation, ein Derivat, oder Fragment davon mit einem natürlichen oder synthetischen cytostatischen oder cytocidalen Agens konjugiert ist.

**14.** Verwendung nach Ansprüchen 12 und 13, dadurch gekennzeichnet, daß der tPA, allele Variationen, Konjugate, Derivate oder Fragmente davon, radioaktiv markiert sind.

**15.** Verwendung nach Anspruch 14, dadurch gekennzeichnet, daß die Radioaktivitätsdosis oberhalb von 500 MBq liegt.

**16.** Verwendung nach einem der Ansprüche 1 bis 15, dadurch gekennzeichnet, daß das Mittel in einer systemisch zu verabreichenden Applikationsform vorliegt.

**17.** Verwendung nach einem der Ansprüche 1 bis 15, dadurch gekennzeichnet, daß das Mittel in einer lokal in das oder an das krankhafte Gewebe zu applizierenden Applikationsform vorliegt.

EP 0 298 436 B1

18. Mittel, welches als wirksame Bestandteile einen tPA, eine allele Variation, ein Derivat, ein Fragment oder ein Konjugat davon enthält und welche mit mindestens einer Markersubstanz verbunden sind, dadurch gekennzeichnet, daß die wirksamen Bestandteile im hauptsächlich aus den tumorbindenden Anteilen bestehen und keine Proteolyseaktivität zeigen.

19. Mittel nach Anspruch 18, dadurch gekennezeichnet, daß der tPA als Fragment in der Größenordnung von 10 kDa vorliegt.

20. Mittel nach Ansprüchen 18 und 19, dadurch gekennzeichnet, daß es als Konjugat mit einem natürlichen oder synthetischen cytostatischen oder cytocidalen Agens vorliegt.

21. Verfahren zur Herstellung eines Mittels für die diagnostische oder prognostische Abklärung von Tumoren, dadurch gekennzeichnet, daß man
   a. ein tPA Molekül, eine allele Variation, ein Derivat oder Fragment davon isoliert oder herstellt,
   b. dieses Material radioaktiv markiert,
   c. den Radioaktivitätsdosisbereich zwischen 10 und 1000 MBq wählt und
   d. das derartig markierte Material in eine für die Szintigraphie geeignete Lösung überführt.

22. Mittel für die diagnostische und prognostische Abklärung von Tumoren, dadurch erhältlich, daß man nach Anspruch 21 gemäß den Schritten a. bis c. verfährt und das erhaltene Material in eine für die Infusion oder Injektion geeignete Lösung überführt.

23. Verfahren zur Herstellung eines Mittels für die therapeutische Behandlung von Tumoren, dadurch gekennzeichnet, daß man
   a. ein tPA Molekül, eine allele Variation, ein Derivat oder Fragment davon isoliert oder herstellt,
   b. dieses Material radioaktiv markiert,
   c. den Radioaktivitätsdosisbereich oberhalb von 500 MBq wählt,
   d. das gemäß Schritt a. oder b. oder c. erhaltene Material gewünschtenfalls mit einem cytotoxisch wirksamen Agens konjugiert und
   e. das radioaktiv markierte und/oder konjugierte Material in eine geeignete wässrige Lösung überführt.

24. Pharmazeutisches Mittel für die therapeutische Behandlung von Tumoren, dadurch erhältlich, daß man nach Anspruch 23 gemäß den Schitten a. bis d. verfährt und das erhaltenen Material in eine für die Infusion oder Injektion geeignete Lösung überführt.

**Claims**

1. Use of tPA, an allelic variant, a derivative or fragment thereof, for preparing an agent for diagnostic and/or prognostic investigation of malignant tumours.

2. Use according to claim 1, characterised in that the tPA contained therein or a fragment thereof are of natural origin and have been isolated from eukaryotic cell cultures.

3. Use according to claim 1, characterised in that the tPA contained therein or a fragment thereof originate from prokaryotic and eukaryotic cells changed by DNA recombination.

4. Use according to claims 1 to 3, characterised in that the tPA or a fragment thereof contained therein consists mainly of the tumour-binding components and has no proteolytic activity.

5. Use according to one of claims 1 to 4, characterised in that the tPA or a fragment thereof contained therein are derivatives changed by controlled mutagenesis which have a characteristic activity or affinity to tumour tissues at least equal to that of the original genuine forms.

6. Use according to one of claims 1 to 5, characterised in that the tPA or a fragment thereof contained therein is obtained from variations caused by multiple alleles or polymorphism.

7. Use according to one of claims 1 to 6, characterised in that the tPA contained therein is an allelic

13

variation, a derivative or fragment thereof which has at least one finger and/or epidermal growth factor domain or consists only of at least one of these N-terminal parts or of combinations of these parts.

8. Use according to one of claims 1 to 7, characterised in that the tPA fragment present is of the order of 10 kDa.

9. Use according to one of claims 1 to 8, characterised in that the tPA or a derivative thereof is radioactively labelled.

10. Use according to one of claims 1 to 9, characterised in that the tPA contained therein, an allelic variation, a derivative or a fragment thereof, are radioactively labelled, particularly with $^{131}$I, $^{123}$I, $^{99m}$Tc, and/or $^{111}$In.

11. Use of tPA, an allelic variation, a derivative, a conjugate or a fragment thereof for preparing pharmaceutical compositions for the therapeutic treatment of malignant tumours.

12. Use according to claim 11, characterised in that the pharmaceutical composition containing the tPA, an allelic variation, a derivative or a fragment thereof is in the form of a conjugate with at least one cytotoxic substance.

13. Use according to claim 12, characterised in that the tPA contained in the composition, an allelic variation, a derivative or fragment thereof is combined with a natural or synthetic cytostatic or cytocidal agent.

14. Use according to claims 12 and 13, characterised in that the tPA, allelic variants, conjugates, derivatives or fragments thereof, are radioactively labelled.

15. Use according to claim 14, characterised in that the dosage of radioactivity is about 500 MBq.

16. Use according to one of claims 1 to 15, characterised in that the agent is present in a form for administration by a systemic route.

17. Use according to one of claims 1 to 15, characterised in that the agent is present in a form for administration locally into or onto the diseased tissue.

18. Agent which contains as active ingredients a tPA, an allelic variation, a derivative, a fragment or a conjugate thereof and which is combined with at least one marker substance, characterised in that the active ingredients consist mainly of the tumour-binding components and show no proteolytic activity.

19. Agent according to claim 18, characterised in that the tPA is present as a fragment of the order of 10 kDa.

20. Agent according to claims 18 and 19, characterised in that it is present as a conjugate with a natural or synthetic cytostatic or cytocidal agent.

21. Process for preparing an agent for the diagnostic or prognostic investigation of tumours, characterised in that
   a. a tPA molecule, an allelic variation, a derivative or fragment thereof is isolated or prepared,
   b. this material is radioactively labelled,
   c. the radioactivity dosage range is selected to be between 10 and 1000 MBq and
   d. the material thus labelled is converted into a solution which is suitable for scintigraphy.

22. Agent for the diagnostic and prognostic investigation of tumours, which can be obtained by proceeding according to steps a. to c. in claim 21 and converting the resulting material into a solution which is suitable for infusion or injection.

23. Process for preparing an agent for the therapeutic treatment of tumours, characterised in that
   a. a tPA molecule, an allelic variation, a derivative or fragment thereof is isolated or prepared,

14

b. this material is radioactively labelled,

c. the radioactivity dosage range is selected to be above 500 MBq,

d. if desired the material obtained according to step a. or b. or c. is conjugated with a cytotoxically active agent and

e. the radioactively labelled and/or conjugated material is converted into a suitable aqueous solution.

24. Pharmaceutical agent for the therapeutic treatment of tumours, which can be obtained by proceeding according to steps a. to d. in claim 23 and converting the resulting material into a solution which is suitable for infusion or injection.

**Revendications**

1. Utilisation du tPA, d'une variation allélique, d'un dérivé ou d'un fragment de celui-ci pour la préparation d'un agent pour la mise en évidence diagnostique et/ou pronostique des tumeurs malignes.

2. Utilisation selon la revendication 1, caractérisée en ce que le tPA contenu ou un fragment de celui-ci est d'origine naturelle et a été isolé à partir de cultures de cellules eucaryotes.

3. Utilisation selon la revendication 1, caractérisée en ce que le tPA contenu ou un fragment de celui-ci provient de cellules procaryotes et eucaryotes modifiées par recombinaison d'ADN.

4. Utilisation selon les revendications 1 à 3, caractérisée en ce que le tPA contenu ou un fragment de celui-ci consiste principalement en les fractions qui se fixent aux tumeurs et ne présente aucune activité de protéolyse.

5. Utilisation selon l'une des revendications 1 à 4, caractérisée en ce que le tPA contenu ou un fragment de celui-ci est un dérivé modifié par mutagenèse dirigée qui présente une activité caractéristique ou une affinité pour les tissus tumoraux au moins identique à celle des formes authentiques d'origine.

6. Utilisation selon l'une des revendications 1 à 5, caractérisée en ce que le tPA contenu ou un fragment de celui-ci consiste en des variations provoquées par allélie multiple ou par des polymorphismes.

7. Utilisation selon l'une des revendications 1 à 6, caractérisée en ce que le tPA contenu est une variation allélique, un dérivé ou un fragment de celui-ci qui présente au moins un domaine en doigt et/ou de facteur de croissance épidermique ou qui consiste uniquement en au moins l'une de ces parties N-terminales ou en combinaisons de ces parties.

8. Utilisation selon l'une des revendications 1 à 7, caractérisée en ce que le fragment de tPA est présent à raison d'environ 10 kDa.

9. Utilisation selon l'une des revendications 1 à 8, caractérisée en ce que le tPA ou un dérivé de celui-ci est marqué.

10. Utilisation selon l'une des revendications 1 à 9, caractérisée en ce que le tPA contenu, une variation allélique, un dérivé ou un fragment de celui-ci, est radiomarqué, en particulier avec $^{131}I$, $^{123}I$, $^{99m}Tc$ et/ou $^{111}In$.

11. Utilisation du tPA, d'une variation allélique, d'un dérivé, d'un conjugué ou d'un fragment de celui-ci pour la préparation de médicaments pour le traitement thérapeutique des tumeurs malignes.

12. Utilisation selon la revendication 11, caractérisée en ce que le médicament contenant le tPA, une variation allélique, un dérivé ou un fragment de celui-ci est sous forme de conjugué avec au moins une substance active du point de vue cytotoxique.

13. Utilisation selon la revendication 12, caractérisée en ce que le tPA contenu dans le médicament, une variation allélique, un dérivé ou un fragment de celui-ci est conjugué avec un agent cytostatique ou cytocide naturel ou synthétique.

15

**14.** Utilisation selon les revendications 12 et 13, caractérisée en ce que le tPA, les variations alléliques, les conjugués, les dérivés ou les fragments de celui-ci, sont radiomarqués.

**15.** Utilisation selon la revendication 14, caractérisée en ce que la dose de radioactivité est supérieure à 500 MBq.

**16.** Utilisation selon l'une des revendications 1 à 15, caractérisée en ce que l'agent est présent sous une forme d'application à administrer de manière systémique.

**17.** Utilisation selon l'une des revendications 1 à 15, caractérisée en ce que l'agent est présent sous une forme d'application à appliquer localement dans ou sur le tissu malade.

**18.** Agent contenant comme composants actifs un tPA, une variation allélique, un dérivé, un fragment ou un conjugué de celui-ci qui sont liés à au moins une substance de marquage, caractérisé en ce que les composants actifs consistent principalement en les fractions qui se fixent aux tumeurs et ne présentent aucune activité de protéolyse.

**19.** Agent selon la revendication 18, caractérisé en ce que le tPA est présent sous forme d'un fragment de l'ordre de 10 kDa.

**20.** Agent selon les revendications 18 et 19, caractérisé en ce qu'il est présent sous forme de conjugué avec un agent cytostatique ou cytocide naturel ou synthétique.

**21.** Procédé de préparation d'un agent pour la mise en évidence diagnostique ou pronostique des tumeurs, caractérisé en ce que
a. on isole ou prépare une molécule de tPA, une variation allélique, un dérivé ou un fragment de celle-ci,
b. on marque radioactivement ce produit,
c. on choisit le domaine de dose de radioactivité entre 10 et 1000 MBq, et
d. on met le produit ainsi marqué sous forme d'une solution convenant pour la scintigraphie.

**22.** Agent pour la mise en évidence diagnostique et pronostique des tumeurs, que l'on peut obtenir en procédant selon la revendication 21 en suivant les étapes a. à c. et en mettant le produit obtenu sous forme d'une solution convenant pour l'infusion ou l'injection.

**23.** Procédé de préparation d'un agent pour le traitement thérapeutique des tumeurs, caractérisé en ce que
a. on isole ou prépare une molécule de tPA, une variation allélique, un dérivé ou un fragment de celle-ci,
b. on marque radioactivement ce produit,
c. on choisit le domaine de dose de radioactivité supérieur à 500 MBq,
d. on conjugue si on le souhaite avec un agent actif du point de vue cytotoxique le produit obtenu selon l'étape a., b. ou c. et
e. on met le produit radiomarqué et/ou conjugué sous forme d'une solution aqueuse appropriée.

**24.** Agent pharmaceutique pour le traitement thérapeutique des tumeurs, que l'on peut obtenir en procédant selon la revendication 23 en suivant les étapes a. à d. et en mettant le produit obtenu sous forme d'une solution convenant pour l'infusion ou l'injection.

Fig.1

Fig. 2

Fig. 3

Fig. 4

Fig.5

Fig. 5